# EUROPEAN PATENT APPLICATION

(11) **EP 1 374 836 A1**
(43) Date of publication of application: **02.01.2004**
(21) Application number: 02713260.4
(22) Date of filing: 29.03.2002
(51) Int. Cl.: A61K 7/06, A61K 33/00, A61P 17/16, A23L 1/30, A01N 59/16

(54) **HAIR-RESTORING LIQUID COMPRISING AQUEOUS DISPERSION OF ULTRAFINE TITANIUM PARTICLES AND PROCESS AND APPARATUS FOR PRODUCING THE SAME**

(30) Priority: 29.03.2001 JP 2001094608
(71) Applicant: Phild Co., Ltd., Kamigyo-ku, Kyoto-city, Kyoto 602-0023 (JP)
(72) Inventor: HIRATA, Y., c/o Phild Co., Ltd., 110, Gosyohachi-, Kamigyo-ku, Kyoto-shi, Kyoto 602-0023 (JP); UEDA, Y., c/o Phild Co., Ltd., 110, Gosyohachi-, Kamigyo-ku, Kyoto-shi, Kyoto 602-0023 (JP); TAKASE, H., c/o Phild Co., Ltd., 110, Gosyohachi-, Kamigyo-ku, Kyoto-shi, Kyoto 602-0023 (JP); SUZUKI, K., c/o Phild Co., Ltd., 110, Gosyohachi-, Kamigyo-ku, Kyoto-shi, Kyoto 602-0023 (JP)
(74) Representative: VOSSIUS & PARTNER
(86) International application number: PCT/JP2002/003185
(87) International publication number: WO 2002/078656

(57) **Abstract**

Produce a dispersion of ultra-fine titanium particles that offers a hair-repairing effect, by burning a mixture gas of oxygen and hydrogen in high-pressure water, using the combustion gas to melt titanium metal, and causing the molten titanium to collide with high-pressure water and letting the produced ultra-fine titanium particles disperse in water.

## Description

### Field of the Invention

This invention relates to a hair-repair liquid comprising dispersed ultra-fine titanium particles.

The invention also relates to a high-function water offering bioactivity, such as a hair-repair liquid containing titanium metal, and a method and apparatus for producing the same.

### Background of the Invention

Titanium metal is a relatively new metal material discovered much more recently than iron, copper and aluminum. The lightweight metal offering excellent strength at high temperature is already used in wide industrial applications, such as jet engine material for aircraft and spaceship, tube and tube plate comprising heat-exchangers used for nuclear or thermal power generation, and articles of daily use such as eyeglass frame and golf club head. The applications of titanium are expected to grow further.

A relatively large number of examples have been known to date regarding the use of titanium metal in articles of daily use, health/medical products and cosmetics. These examples include barber's scissors with titanium film coatings (Japanese Patent Application Laid-open No. 62-268584), the utilization of far-infrared ray through molten titanium metal (Japanese Patent Application Laid-open Nos. 61-59147, 1-155803 and 3-112849), bedding (Japanese Patent Application Laid-open No. 8-322695), cooking utensil (Japanese Patent Application Laid-open No. 9-140593), eye mask (Japanese Patent Application Laid-open No. 10-71168), health-maintaining equipment (Japanese Patent Application Laid-open Nos. 11-285541 and 11-285543), health band (Japanese Utility Model Registration No. 3045835) and health slippers (Japanese Utility Model Registration No. 3061466).

The use of titanium compounds, particularly titanium oxides, for hair treatment purposes such as hair dyes, hair cosmetics and hair-protecting agents is also known. The examples in this field include an oxide-dye composition containing titanium oxide (Japanese Patent Application Laid-open No. 57-18606), hair cosmetic containing titanium oxide, silicon oxide, zirconium oxide or polymeric silicone composition (Japanese Patent Application Laid-open Nos. 63-270620 and 1-113313), hair cosmetic made of water-insoluble silicone containing titanium mica (Japanese Patent Application Laid-open No. 1-224310), UV-protecting agent for hair containing fine titanium particles (Japanese Patent Application Laid-open Nos. 63-270620 and 5-170635; Published Japanese Translation of PCT International Patent Application Nos. 6-501963 and 6-510747) and hair-coloring composition containing titanium oxide and coloring mica (Japanese Patent Application Laid-open No. 11-139946). However, the hair-repairing effect of a liquid containing titanium metal was not known. On the other hand, an apparatus that uses titanium metal in the cathode to produce drinking water of high alkali-ion content is also known (Japanese Patent Application Laid-open No. 50-40779). However, this apparatus has nothing to do with a hair-repair liquid containing titanium metal.

Application of the almost infinite utilities of titanium in the production of bioactive materials, food materials, medical products, etc., is generating significant interests, but no successful applications have yet to be reported, let alone revelations of the true utilities of titanium.

### Summary of the Invention

The present invention found that the bioactivity of titanium metal could be utilized in the form of a micro-dispersion of titanium metal in the production of various bioactive materials, health/medical products, and in particularly, hair-repair liquids offering highly beneficial repairing effects.

The purpose of the present invention is to provide a hair-repair liquid, as well as a method and apparatus for producing the same, wherein the hair repair liquid is made of high-function water, which is produced by melting titanium metal with a combustion gas of hydrogen and oxygen in high-pressure water, causing the molten titanium to collide with high-pressure water, and then micro-dispersing the obtained ultra-fine titanium particles.

In the context of the present invention, "titanium micro-dispersion water" refers to water in which ultra-fine titanium particles are micro-dispersed and a small amount of titanium is dissolved.

The present invention is characterized by a liquid for hair repair and other applications, as well as a method and apparatus for producing the same, wherein the liquid for hair repair and other applications is made of a high-function water in which ultra-fine titanium particles are dispersed, which is produced by melting titanium metal in high-pressure water with the heat generated from burning a mixture gas of oxygen and hydrogen. Specifically, the present invention is defined by components (1) through (4) below:
(1) A hair-repair liquid made of titanium micro-dispersion water in which ultra-fine titanium particles are dispersed.
(2) A method for producing a hair-repair liquid made of titanium micro-dispersion water in which ultra-fine titanium particles are dispersed, wherein the method is characterized by burning a mixture gas of oxygen and hydrogen in high-pressure water, melting titanium metal using the combustion gas, and then dispersing ultra-fine titanium particles in high-pressure water.
(3) An apparatus for producing a hair-repair liquid made of titanium micro-dispersion water in which ultra-fine titanium particles are dispersed, wherein the apparatus consists of a pressure-resistant container comprising a high-pressure water tank, an injector nozzle for mixture gas of oxygen and hydrogen, a titanium metal bar, an ignition device and a combustion chamber.
(4) An apparatus for producing a hair-repair liquid made of titanium micro-dispersion water in which ultra-fine titanium particles are dispersed, as described in (3) above, wherein the apparatus has as an adjunct a water electrolyzer for producing a mixture gas of oxygen and hydrogen.

It has been known that generally in a water dispersion or mixed solution in which fine titanium particles or titanium oxide particles are simply dispersed, the titanium content precipitates and separates in a short period. The most important characteristic of the titanium micro-dispersion water obtained by the present invention is that the titanium melted by combustion heat via the aforementioned means exists as ultra-fine particles micro-dispersed in high-pressure water and therefore remains stable for a long time without precipitating or separating. This micro-dispersion water also contains a small amount of dissolved titanium.

As explained above, the titanium micro-dispersion water obtained by the present invention provides an unexpected but significant benefit as a hair-repair liquid made of high-function water in which water molecules and ultra-fine titanium particles interact with each other to produce bioactivity.

In general, hair is damaged by external irritations such as friction, heat from the sun, perm chemicals and inappropriate cuts, as well as by malnutrition caused by an unbalanced diet or drastic weight loss. Since damaged hair has no self-repairing ability, it must be protected externally to prevent further damage. Treatment products are used for this purpose. As is the case with the skin, water and oil are the most important constituents of the hair. In particular, water is absorbed into the hair cortex, which is a component of hair, and adds moisture and a supple look to the hair while making the hair more flexible so as to let it bounce as the body moves. Oil forms an oil film over the hair cuticle and adds shine and gloss to the hair while minimizing friction and preventing damage to the hair cuticle.

The high-function titanium micro-dispersion water obtained by the present invention has been confirmed by the trial test explained later to provide excellent treatment effects not achieved by other commercial treatment products available today. The water indeed has a significant repair effect on damaged hair.

In addition to hair-repair liquids, the high-function titanium micro-dispersion water obtained by the present invention can also be used in health products such as motor-function enhancing creams, medical products such as antibacterial agents, UV-protection products, healthy drinking water, health supplements, cosmetics, food preservatives, freshness-keeping agents for food, insect repellents, deodorants, and so on.

The titanium micro-dispersion water obtained by the present invention can be used suitably for the above purposes today, but it is firmly believed that this micro-dispersion water will provide an innovative bioactive material that would fully answer the needs of today's health-conscious consumers.

The mechanism as to why a micro-dispersion water of ultra-fine titanium particles provides excellent bioactive functions is not yet clear. The inventors are working diligently to find scientific explanations for these functions.

### Brief Description of the Drawings

Fig. 1: Production flow chart of a hair-repair liquid made of titanium micro-dispersion water as proposed by the present invention
Fig. 2: Schematic drawing of an apparatus for producing a hair-repair liquid made of titanium micro-dispersion water as proposed by the present invention
Fig. 3: Certificate showing the analysis test result of titanium micro-dispersion water as obtained by the present invention
Fig. 4: Microphotographs sowing the conditions of hairs treated with titanium micro-dispersion water as obtained by the present invention and with water

### Description of the Symbols

1: Apparatus for producing titanium micro-dispersion water as proposed by the present invention
2: Pressure-resistant container for production of titanium micro-dispersion water
3: Electrolyzer/material-gas generator
5: High-pressure water tank
6: Combustion chamber
7. Pressure control valve
8: Titanium micro-dispersion water outlet
9. High-pressure water
10: Titanium metal bar
11: Ignition device
12: Molten titanium metal
13: Feed cylinder
14: Mixture-gas injector nozzle
16: Hydrogen feed line
17: Oxygen feed line
18: Electrode
18': Electrode
19: Partition
20: Water

### Best Mode for Carrying Out the Invention

A hair-repair liquid made of titanium micro-dispersion water as obtained by the present invention is a new product not heretofore available. The present invention also provides a new method and apparatus for producing a hair-repair liquid made of water that contains titanium as ultra-fine particles, wherein the production of the titanium micro-dispersion water is based on melting titanium metal not through general methods like thermal melting, arc discharge and laser irradiation that are commonly used to melt metal, but by melting it using the heat generated from burning oxygen and hydrogen, and then dispersing the resulting ultra-fine titanium particles in high-pressure water.

Specifically, after studying ways to efficiently and economically produce a hair-repair liquid made of titanium micro-dispersion water, as well as ways to apply the titanium micro-dispersion water to bioactivation purposes, the inventors conceptualized a method to bum hydrogen and oxygen, insert a pure titanium metal bar into the atmosphere and heat it, thereby causing the produced molten titanium to collide with high-pressure water and letting ultra-fine titanium particles disperse in water, wherein a device is incorporated so that hydrogen and oxygen are burned in high-pressure water in order to suppress production of substances other than water and titanium metal.

The method proposed by the present invention requires that the amounts of hydrogen and oxygen to be burned, as well as reaction pressure and feed rate of titanium metal, be controlled in order to add excellent bioactivity to the product. In the aforementioned production method, an appropriate filter system is also necessary because not only ultra-fine titanium particles but also a small amount of fine titanium oxide particles will be produced in water.

A method for producing a hair-repair liquid made of titanium micro-dispersion water as obtained by the present invention in water under high pressure, as well as an apparatus to embody this method, are explained according to the drawings.

Fig. 1 is a production flow chart for hair-repair liquid made of titanium micro-dispersion water as proposed by the present invention. Fig. 2 shows an apparatus for producing a hair-repair liquid made of titanium micro-dispersion water as proposed by the present invention.

The apparatus for producing titanium micro-dispersion water (1) proposed by the present invention, as shown in Fig.2, consists of a pressure container (2) used for producing high-pressure water in which molten titanium is dispersed, an electrolyzer/material-gas generator (3) and a filter system to filter titanium micro-dispersion water (not illustrated).

The basic structural components of the pressure-resistant container (2) used in the present invention comprises a high-pressure water tank (5), an injector nozzle for mixture gas of oxygen and hydrogen (14), a combustion chamber (6) and a titanium metal bar (10), which together work to produce titanium micro-dispersion water in which ultra-fine titanium particles are dispersed. A water electrolyzer (3) for supplying material hydrogen and oxygen and a filter system to filter titanium micro-dispersion water are also attached as adjuncts.

The pressure-resistant container (2) used in the present invention consists of a high-pressure water tank (5) made of metal, or preferably steel. Inside this high-pressure water tank (5), a mixture gas of hydrogen and oxygen generated in the electrolyzer (3) and supplied via hydrogen and oxygen feed lines (16, 17), respectively, is blown out into the combustion chamber (6) through the injector nozzle (14). The titanium metal bar (10) is gradually fed into the combustion chamber (6) from a feed cylinder (13) in accordance with the melt amount. The mixture gas of hydrogen and oxygen is ignited by an ignition device (11) and molten titanium metal is released into high-pressure water (9). Molten titanium collides with high-pressure water and breaks up into ultra-fine particles, and the high-pressure water (9) containing these ultra-fine titanium particles is taken out from an outlet (8) at the bottom of the high-pressure water tank and then filtered through an appropriate filter system (not illustrated).

It is possible to use high-pressure cylinders containing hydrogen and oxygen, instead of a material-gas generator (3), but the oxygen and hydrogen supplied via water electrolysis are completely pure gases. Therefore, using an electrolyzer has the advantage of being able to efficiently supply fuel gases with a theoretical oxygen-to-hydrogen mixture ratio of 1 to 2.

The present invention provides an example of generating hydrogen and oxygen, as the material for producing titanium micro-dispersion water, through electrolysis of water (20) in a material-gas generator (3). (18) and (18') indicate the cathode and the anode, respectively. As described above, it is certainly possible to supply hydrogen and oxygen directly from the respective storage cylinders into the high-pressure water tank (5).

In the apparatus proposed by the present invention, hydrogen and oxygen generated via electrolysis and supplied through the hydrogen and oxygen feed lines (16, 17), respectively, are blown out into the combustion chamber (6) by a pump through the injector nozzle (14). The mixture gas is completely burned to achieve a perfect combustion state leaving an ultrahigh-temperature steam gas. A pure titanium metal bar (10) is inserted into this combustion gas where the bar is heated and melted. The titanium metal bar is inserted into the cylinder (13) at a constant rate in accordance with the melt amount. When melting titanium metal, the hydrogen-to-oxygen mixture ratio must be controlled precisely to 2 to 1. Additionally the pressure inside the high-pressure water tank must be adjusted using a pressure control valve (7).

Molten titanium (12), generated by heating to high temperature and thereby melting titanium metal in the combustion chamber (6), is released from the combustion chamber (6) into high-pressure water (9), where it collides with high-pressure water to produce ultra-fine particles. At this time, a part of titanium is likely to change to a crystalline structure.

Thus ultra-fine titanium particles were produced in water. These titanium particles have a very strong hydrophobic property and are therefore dispersed stably in water. They will not precipitate even when a coagulant is added.

According to the production method proposed by the present invention, to produce one ton of titanium micro-dispersion water ideally a mixture gas under a pressure of approx. 3.5 atmospheres should be injected at a speed of approx 4 to 6 litters per second into the high-pressure water tank containing water compressed to approx. 1.5 to 2.5 atmospheres. If the gas pressure is too high, the apparatus structure may be damaged. If the pressure is low, the gas floats up from the combustion chamber and the heated/melted titanium will be encapsulated in air bubbles and diffused from the water surface. This will reduce the production efficiency of a micro-dispersion of ultra-fine titanium particles.

As for the operation of this apparatus, high-pressure hydrogen and oxygen are injected via a pump into the high-pressure water tank (5) through the nozzle (14) and ignited by the ignition device (11) to obtain a combustion steam gas of ultrahigh temperature. A titanium metal bar (10) is gradually inserted into this combustion gas to melt the bar.

In this apparatus, hydrogen and oxygen must be burned in water to suppress production of substances other than water and ultra-fine titanium particles. Combustion must take place under high pressure in order to burn hydrogen and oxygen in water via pure reaction without allowing impurities to be mixed in. The position to insert the titanium metal bar must be set inside an area where the mixture gas will burn completely and become a perfect steam gas of ultrahigh temperature.

Other characteristics of the present invention includes the use of thus produced titanium micro-dispersion water, in which ultra-fine titanium particles are dispersed, as a material for hair-repair liquids and other health products, cosmetics, food materials, drugs and quasi-drugs after necessary refining. The produced titanium micro-dispersion water contains a small amount of fine titanium-oxide particles generated via bonding of titanium and oxygen and must therefore be filtered and refined as necessary.

So as not to remove the produced ultra-fine titanium particles more than necessary, filtering should preferably be implemented not by using ion exchange or reverse-osmosis membrane, but by using a hollow-fiber membrane system that can yield a desired titanium micro-dispersion water. Specifically, filtering the high-pressure water discharged from the high-pressure water tank sequentially through hollow-fiber membranes is beneficial in terms of enhancing the characteristics of titanium micro-dispersion water and extending the filter life, and such filter system will provide a titanium micro-dispersion water that can meet the required standards governing food and sanitation, cosmetics and drugs.

Fig. 3 shows the analysis result of titanium micro-dispersion water as obtained by the present invention.

Fig. 4 shows the surface condition of hair treated with dispersion water of ultra-fine titanium particles as obtained by the present invention.

In Fig. 4, the photographs on the left show the surface condition of a hair treated with dispersion water of ultra-fine titanium particles as obtained by the present invention. The top photograph was taken at 700 magnifications, while the bottom photograph was taken at 1,000 magnifications. The photographs in the center show the surface condition of a hair treated with distilled water, with the top and bottom photographs taken at 700 and 1,200 magnifications, respectively. The photographs on the right show the surface condition of an untreated hair, with the top and bottom photographs taken at 700 and 1,200 magnifications, respectively.

### "Example"

An embodiment of the present invention is explained in details using an example. Note, however, that the invention is not limited to this example.

Fig. 2 shows a representative example of the production apparatus proposed by the present invention. The figure illustrates an apparatus for producing a hair-repair liquid made of titanium micro-dispersion water in which ultra-fine titanium particles are dispersed, wherein the apparatus consists of a high-pressure water tank (5), an injector nozzle for mixture gas of oxygen and hydrogen (14) and a titanium metal bar (10), and wherein the ultra-fine titanium particles are produced by a collision of molten titanium with high-pressure water.

The high-pressure water tank (5) is a metallic pressure-resistant tank capable of withstanding extra high pressure. A mixture gas of hydrogen and oxygen supplied through hydrogen and oxygen feed lines (16, 17), respectively, is injected into a combustion chamber (6) from the injector nozzle (14), and the titanium metal bar (10) is fed into the combustion chamber via a cylinder (13). The pressure inside the high-pressure water tank (5) must be controlled via a pressure control valve (7). The mixture gas is ignited by an ignition device (11) and then molten titanium (12) is released into high-pressure water, where the titanium collides with high-pressure water and breaks up into ultra-fine titanium particles. Thus produced titanium micro-dispersion water in which ultra-fine titanium particles are dispersed is taken out from an outlet (8).

As for the operation of this apparatus, hydrogen and oxygen are supplied under high pressure and their mixture gas is injected into the high-pressure water tank (5) via the nozzle (14). The mixture gas is ignited by the ignition device (11) and burned completely to obtain a perfect combustion state leaving an ultrahigh-temperature steam gas. The titanium metal bar (10) is inserted into this combustion gas and melted. Molten titanium (12), produced by heating to high temperature and thus melting titanium metal inside the nozzle, is released into high-pressure water, where it collides with high-pressure water and breaks up into ultra-fine particles. At this time, it is considered that a part of titanium changes to a crystalline structure and titanium atoms rearrange themselves accordingly to create a near spherical shape, thus allowing the grain to reach an energy-stable state.

In the produced titanium micro-dispersion water, titanium remains micro-dispersed in water without adding an active agent. The produced titanium micro-dispersion water is discharged from the outlet and sent to a filter system as necessary. The filter system filters the dispersion as it sequentially passes through filter membranes of 50 microns, 25 microns, 3 microns, 0.5 micron and 0.1 micron. Finally, titanium micro-dispersion water is produced that contains a specified amount of ultra-fine titanium particles and a trace amount of dissolved titanium.

### Production Conditions (Example)

Internal pressure of production tank: 2 atmospheres
Mixture gas: 5 L/sec (3.5 atmospheres)
Feed rate of titanium metal: 0.5 kg/2 hrs
Production volume of titanium micro-dispersion water: Approx. 1,000 kg

A hair-repair liquid made of titanium micro-dispersion water in which ultra-fine titanium particles are dispersed was obtained under the above conditions.

The produced titanium micro-dispersion water will be passed through a filter system (consisting of hollow-fiber membranes of 50 microns, 25 microns, 3 microns, 0.5 micron and 0.1 micron positioned sequentially), as necessary.

### "Use Example"

Trial Test of Hair-Repair Liquid Made of Titanium Micro-Dispersion Water:

Ten male and female subjects were instructed to carry out a hair treatment regimen using titanium micro-dispersion water obtained by the present invention in which ultra-fine titanium particles are dispersed, and the effects and efficacies of the treatment were verified.

Using a hair-repair liquid made of titanium micro-dispersion water as obtained under the above production conditions, the test was conducted where the subjects were instructed to wash their hair with the hair-repair liquid as well as spray and apply the liquid to their hair.

Use Conditions and Test Results
- Use conditions: : Washing hair in a basin (3 liters per wash)
- : Spraying onto hair with a spray (10 cc per application)
- : Applying onto hair using a brush (10 cc per application)

1. 10 male and female subjects (The subjects used the liquid at varying frequencies)
2. Efficacies

| | |
|---|---|
| Hair became less tangling | 5 persons |
| Fallen hair became less | 8 persons |
| Hair became shiner | 7 persons |
| Hair became darker | 1 person |
| Hair became denser | 6 persons |
| Scalp became smoother | 7 persons |
| Dandruff became less | 7 persons |

As evident from the above test results, many of those who washed their hair with the titanium micro-dispersion water obtained by the present invention or applied/sprayed the micro-dispersion to their hair cited recovery of damaged hair, improved hair condition, smoother scalp, reduced dandruff and other improvements. Therefore, it is found that the micro-dispersion will have excellent effects on human hair/skin through products that come in contact with human hair/skin.

### Industrial Field of Application

The present invention, whose structure is explained above, provides high-function water made of newly developed titanium micro-dispersion water, as well as its production method and apparatus. The titanium micro-dispersion water obtained by the present invention contains ultra-fine titanium particles in a dispersed state and therefore exhibits high bioactivity, and test has confirmed the excellent hair-repairing effect of this micro-dispersion.

Besides hair-repair liquids, the high-function water made of titanium micro-dispersion water as obtained by the present invention can be used in the production of healthy drinking water, health supplements, cosmetics, food preservatives, freshness-keeping agents for food, insect repellents and deodorants.

## Claims

1. A hair-repair liquid comprising dispersion water in which ultra-fine titanium particles are dispersed.

2. A method for producing a hair-repair liquid comprising dispersion water in which ultra-fine titanium particles are dispersed, said method comprising burning a mixture gas of oxygen and hydrogen in high-pressure water and using the burning gas to melt titanium metal, thereby dispersing the resultant ultra-fine titanium particles in water.

3. An apparatus for producing a hair-repair liquid comprising dispersion water in which ultra-fine titanium particles are dispersed, said apparatus comprising a pressure-resistant container comprising a high-pressure water tank, an injector nozzle for mixture gas of oxygen and hydrogen, a titanium metal bar, an ignition device and a combustion chamber.

4. The apparatus for producing a hair-repair liquid as described in Claim 3, comprising a water electrolyzer to produce a mixture gas of oxygen and hydrogen.

5. Health products, medical products such as antibacterial agents, UV-protection products, healthy drinking water, health supplements, cosmetics, food preservatives, freshness-keeping agents for food, insect repellents or deodorants made from titanium micro-dispersion water in which ultra-fine titanium particles are dispersed.
